# EUROPEAN PATENT APPLICATION

(11) **EP 1 024 357 A2**
(43) Date of publication of application: **02.08.2000**
(21) Application number: 00300671.5
(22) Date of filing: 28.01.2000
(51) Int. Cl.: G01N 27/06, G01N 27/416

(54) **Detection means**

(30) Priority: 29.01.1999 GB 9902087
(71) Applicant: The BOC Group plc, Windlesham Surrey GU20 6HJ (GB)
(72) Inventor: Baker, Derek Martin, Keynsham, Bristol BS3 1TB (GB)
(74) Representative: Bousfield, Roger James

(57) **Abstract**

A process for the detection of water soluble noxious substances in a gas/vapour stream which comprises contacting the gas/vapour stream with a small volume of water and measuring the subsequent conductivity and/or pH changes over time wherein when the aqueous solution containing the dissolved noxious gas stream as aquated ions exceeds a pre-determined level of conductivity and/or pH the solution is cleansed in situ of the noxious substances to render it suitable for further use.

## Description

This invention relates to an assembly for the detection and quantification of noxious substances from exhaust gas streams, particularly those found in the semiconductor industry.

Large quantities of noxious substances are employed in the semiconductor industry in the processing of electronic wafers/chips and other devices. These includes any gases vapour or fine dusts that are partially or wholly water soluble, for example HCl, Cl₂, BCl₃, HF, F₂, NH₃, Cl₂SiH₂, ClF₃, WF₆, CO₂, AlCl₃; such gases/fumes are either acidic or basic. They are toxic and/or corrosive and/or pyrophoric and their undetected release from semiconductor processing chambers could lead to a number of problems including blocking the chamber ducts, fire hazards, corrosion of the processing apparatus and a possible toxic gas/vapour release in to the workspace. In addition, the release of these substances could occur in untreated exhaust gas streams or through a breach in the processing containment or through the exhaustion or failure of a suitable scrubbing device designed to remove or reduce these noxious substances from the exhaust gas stream to an environmentally safe concentration.

Detection of these noxious substances can be achieved in a variety of ways, for example, mass spectroscopy, gas chromatography, infra-red spectroscopy, electrochemical cell detection, paper tape detection, solid state sensor detection and quantitative chemical indicator sampling tube detection.

A further method of detection is to monitor the effect that these substances have on the conductivity and/or pH of liquid water (H₂O) by contacting these substances with water. On contact with water some part or all of the substance(s) dissolves in the water. This causes a change in electrical conductivity and/or pH which may be measured with a suitable conductivity and/or pH cell. If sampling in to a non-renewed body of water, the conductivity and/or pH would rise until the body of liquid had been saturated with the noxious substance(s) which may precipitate out as salts and/or the body of water will evaporate to dryness; a renewable water source is therefore desirable.

The design of the interface between the noxious substance and the body of water is of great importance to the sensitivity and/or speed of response of such detectors. Both are enhanced by using the smallest volume of water (<200cm³) and the largest volume of noxious gas practical to the application. Restricting the design in this way makes packaging the conductivity and/or pH cell problematic as both need a minimum volume of water to work in effectively. The design of the assembly must also be practical in that it must be able to be sited suitably close to the possible source of noxious substance(s).

Traditional designs of detection equipment relying on changes in conductivity and/or pH of a body of water in contact with a noxious gas stream do not possess a renewable water supply and feature a large volume of water (>200cm³). This restricts the sensitivity and response time of the detector making it unsuited to the detection of chemical processes occurring in the processing chambers over a timescale of several minutes.

There is therefore a need for an improved detection assembly of the type which relies on conductivity and/or pH changes to detect noxious substances.

In accordance with the invention, there is provided a process for the detection of water soluble noxious substances in a gas/vapour stream which comprises contacting the gas/vapour stream with a small volume of water and measuring the subsequent conductivity and/or pH changes over time, wherein when the aqueous solution containing the dissolved noxious gas stream as aquated ions exceeds a pre-determined level of conductivity and/or pH the solution is cleansed in situ of the noxious substances to render it suitable for further use.

To achieve this, the solution is preferably passed through an ion-exchange bed and retained for further use.

The noxious gas stream can be contacted with the water by either pumping it through the water, or by exposing a surface area of the water to the noxious gas, and latterly measuring the resulting liquor with the conductivity or pH, probe; the term pH herein includes pIon measurements.

The ion exchange bed preferably comprises an ion exchange resin of known general type. The ion-exchange resin serves to remove or scrub the ions from the water and return it to its initial de-ionised state, typically with a conductivity of less than 5 micro-siemens/cm. The water can then be reused to detect noxious gases/vapours in the same way as already outlined. Advantageously, the initial small volume of water is de-ionised also.

Any soluble or partially soluble gas/vapour may be detected by this method. The noxious gases/vapour substance may comprise one or more of a halide, a mineral acid gas, a silicon halide, especially a silicon tetrahalide and a boron trihalide.

Specific examples of gases/vapours which can be dissolved & detected are HCl, Cl₂, HF, F₂, NH₃, Cl₂SiH₂, ClF₃, WF₆, and CO₂.

The unit may be calibrated to give quantitative information on the concentration in parts per million (ppm) of which ever gas/vapour detection is required simply by selecting the appropriate gas/vapour on the alpha-numeric display which is connected to the detection apparatus through a programmable logic controller (PLC) interface.

This assembly may be employed to detect noxious gas or vapour substances in gas streams which comprise the substance(s) as either a pure gas/vapour or ones which are diluted in some inert water insoluble gas such as nitrogen, argon and/or helium which are commonly used purge and/or diluent gases. Sampling from the atmosphere will generally lead to spurious results due to the presence of carbon dioxide which is partially soluble in water and detectable by both conductivity and pH cells.

By sampling relatively large volumes of gas (<500sccm) in to small volumes of liquid (<200cm³) and by using extremely sensitive conductivity (K=0.1, 1.0) and/or pH cells it is possible to determine very small changes in the conductivity and/or pH/pIon. If the volume of liquid being used is known and fixed, if the noxious gas/vapour sample rate is known and fixed and if the gas/vapour is known and fixed and the sampling time is known, then the concentration of the noxious gas/vapour can be accurately determined to the order of parts per million (ppm). The PLC control may be programmed to respond to incremental changes of conductivity and or pH to give an 'instant' response to changing levels of noxious gas/vapour and/or the PLC may give an average indication of the concentration by responding to specific (and larger) changes in conductivity and/or pH over a longer period of time.

The PLC control of the assembly may include the option to switch electrically and/or mechanically an external body in response to the calculated concentration of the noxious gas stream, for example to direct the noxious substance detected to scrubber and/or collection means.

The assembly will have the ability to increase or decrease the noxious gas sampling rate in response to its concentration. For example, if the PLC detects a low concentration noxious gas stream, the sampling rate could be increased to increase the speed of detection.

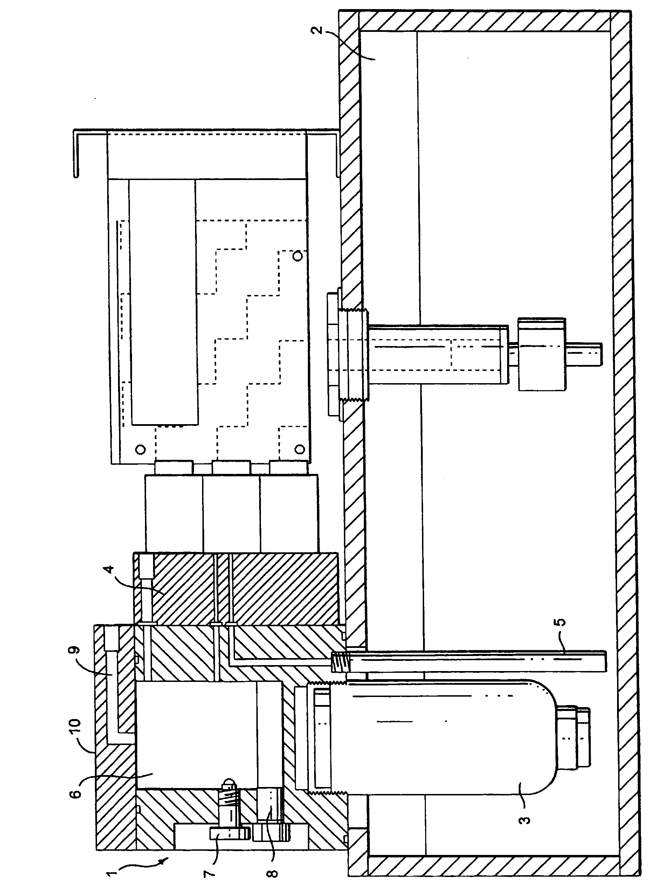

For a better understanding of the invention, reference will now be made, by way of exemplification only, to the accompanying drawing which shows a schematic representation apparatus used in the invention.

With reference to the drawing there is shown an apparatus which the process of the invention can be put in to effect. The apparatus comprises a conductivity cell generally indicated at 1, a water reservoir 2, an ion exchange resin cartridge 3 and a solenoid actuated valve manifold 4.

In operation of the apparatus, de-ionised water is moved from the water reservoir 2 under the action of compressed nitrogen (<2psia) above the water level in the reservoir 2 in to the water fill tube 5 and, via the valve manifold 4, in to a cell chamber 6 of the conductivity cell 1. A water level sensor 7 is present in the chamber 6 to limit the amount of water thus introduced to the height of the sensor. The reservoir 2 is then vented to atmosphere.

A conductivity probe 8 at the base of the chamber 6 takes a measurement with an apparatus programmed controller deciding whether to keep or to reject the water sample in the chamber 6 in accordance with pre-determined criteria. Such criteria can include whether the conductivity of the water is below a threshold value - if so, sampling ban begin; or whether the conductivity is above the threshold - if so, the water needs cleansing as described below.

If the conductivity of the water allows sampling to begin, the apparatus controller initiates sampling of noxious gases/vapours in an exhaust stream pumped in to the chamber 6 from a gaseous source and via a bore 9 in a lid 10 of the chamber 6.

The conductivity probe 8 continually measures the changes in conductivity of the water in the chamber 6 caused by the presence of the gas exhaust stream. In particular, if the conductivity of the water starts to increase, this shows that a water soluble gas is present. The apparatus controller is programmed to interpret the conductivity results - usually including data in respect of predetermined species in (or arising from) the exhaust gas stream - and display results, for example the concentration of the exhaust stream species in parts per million (ppm) and/or indicate by an alarm if the concentration exceeds certain pre-determined limits. The apparatus controller decides when sampling can cease and the water in the chamber 6 should be renewed. When this decision has been made, pumping of the exhaust gas stream is terminated and the apparatus controller introduces pressurised nitrogen (2psi) in to the manifold for and simultaneously opens a valve (not shown) for a fixed period in order to release the contaminated water by gravity and the nitrogen gas pressure in to ion exchange resin in the cartridge 3 and thence in to the water reservoir 2 as cleansed, de-ionised water.

The sequence is repeated until the conductivity probe indicates to the apparatus controller by means of high conductivity measurements that the ion-exchange resin is exhausted and therefore needs replacing and/or a minimum water level indicator 11 indicates a low water level and that the reservoir therefore needs refilling with new de-ionised water. Under either of these two conditions, the apparatus user is warned by the apparatus controller by visual and/or audible signs to take corrective action.

In this embodiment of the invention shown in the drawing, and generally, the conductivity probe may be replaced by a pH/pIon probe and the apparatus modified accordingly.

## Claims

1. A process for the detection of water soluble noxious substances in a gas/vapour stream which comprises contacting the gas/vapour stream with a small volume of water and measuring the subsequent conductivity and/or pH changes over time wherein when the aqueous solution containing the dissolved noxious gas stream as aquated ions exceeds a pre-determined level of conductivity and/or pH the solution is cleansed in situ of the noxious substances to render it suitable for further use.

2. A process according to Claim 1 or Claim 2 in which the solution is cleansed by passing it through an ion-exchange bed.

3. A process according to Claim 1 or Claim 2 in which the noxious gas stream is pumped through the water.

4. A process according to any preceding claim in which the noxious gas stream is exposed to a surface of the water.

5. Apparatus for carrying out the process of any one of Claims 1 to 4 including a water reservoir, an ion-exchange bed, a chamber for receiving water from the reservoir and having a probe for electrical conductivity/pH measurement of the water and means for returning the water to the reservoir via the ion-exchange bed.
